Europäisches Patentamt

European Patent Office

Office européen des brevets

(11) Numéro de publication : **0 288 382 B1**

# (12) FASCICULE DE BREVET EUROPEEN

(45) Date de publication du fascicule du brevet :
**29.07.92 Bulletin 92/31**

(51) Int. Cl.⁵ : **C08B 37/00, A61K 31/715, A61K 39/39**

(21) Numéro de dépôt : **88400963.0**

(22) Date de dépôt : **20.04.88**

(54) **Nouveau dérivé de D.25, procédé de préparation, utilisation à titre d'agent immunostimulant et compositions pharmaceutiques le contenant.**

(30) Priorité : **22.04.87 FR 8705690**

(43) Date de publication de la demande :
**26.10.88 Bulletin 88/43**

(45) Mention de la délivrance du brevet :
**29.07.92 Bulletin 92/31**

(84) Etats contractants désignés :
**AT BE CH DE ES FR GB GR IT LI LU NL SE**

(56) Documents cités :
**EP-A- 0 113 123**
**PATENT ABSTRACTS OF JAPAN, vol. 10, no. 28 (C- 326)[2085], 4 février 1986; & JP-A-60 181 020 (YUKIJIRUSHI NIYUUGIYOUK.K.) 14-09-1985**

(56) Documents cités :
**CARBOHYDRATE RESEARCH, vol. 49, 1976, pages 335-340, Elsevier Scientific Publishing Co., Amsterdam, NL; W. JACK et al.: "Theoxidation of terminal D-galactofuranose residues of a galactan and a glycoprotein by a D-galactose oxidase preparation fromDactylitum dendroides"**

(73) Titulaire : **PIERRE FABRE MEDICAMENT**
**45, Place Abel Gance**
**F-92100 Boulogne (FR)**

(72) Inventeur : **Dussourd d'Hinterland, Lucien**
**Domaine de Plombière**
**F-81100 Castres (FR)**
Inventeur : **Normier, Gérard**
**23 rue Francis Poulenc**
**F-81100 Castres (FR)**
Inventeur : **Pinel, Anne-Marie**
**22 rue des Capucins**
**F-81100 Castres (FR)**

(74) Mandataire : **Ahner, Francis et al**
**CABINET REGIMBEAU, 26, avenue Kléber**
**F-75116 Paris (FR)**

## Description

La présente invention concerne de nouveaux composés dérivés du D.25, leur procédé de préparation et des compositions pharmaceutiques les contenant.

Le produit dénommé D.25 est le polysaccharide extrait des protéoglycanes membranaires bactériens, composé essentiellement d'unités galactose et ayant un poids moléculaire de $30 \pm 10$ KD. Ce polysaccharide a été décrit en détail dans le brevet français n° 84.13844 déposé le 10 Septembre 1984. Ce polysaccharide possède des propriétés immunostimulantes, notamment vis-à-vis de l'induction d'interféron endogène et de l'activation des cellules NK (Natural Killer). Ce polysaccharide est préférentiellement isolé d'une souche non capsulée et non pathogène de Klebsiella pneumoniae biotype a, déposée à la Collection Nationale de l'Institut Pasteur sous le numéro 145.I.IP.

La présente invention concerne un composé dérivés du D.25 dans lesquels tous les résidus galacto-furannose ($Gal_f$) de la chaîne polysaccharidique linéaire du D.25 ont été transformés en arabinose, sans aucune autre modification du produit initial. Dans ce composé, les résidus galacto-pyranose ($Gal_p$) sont conservés.

Ce composé, est défini par la monomère suivant :

$$\xrightarrow[1,3]{} \beta\ Gal_p \xrightarrow[1,3]{} \beta\ Ara \xrightarrow[1,3]{} \alpha\ Gal_p \xrightarrow[1,3]{} \beta\ Gal_p$$

$$\xrightarrow[1,3]{} \alpha\ Ara \xrightarrow[1,3]{} \alpha\ Gal_p \xrightarrow[1,3]{} \alpha\ Ara \xrightarrow[1,3]{} \beta\ Gal_p \xrightarrow[1,3]{} \alpha\ Gal_p$$

$$\xrightarrow[1,3]{} \beta\ Ara \xrightarrow[1,3]{}$$

dans lequel $Gal_p$ signifie galacto pyranose (formes $\alpha$ et $\beta$)

Ara signifie arabinose (formes $\alpha$ et $\beta$)

L'invention concerne également des dérivés des composés précédents, à savoir notamment :
– des dérivés d'hémisynthèse
– des dérivés marqués
– des dérivés conjugués.

Parmi les dérivés d'hémisynthèse, il faut citer les amides, les esters, les éthers, les sels ou dérivés d'ammonium quaternaire avec des acides, amines, amides ou alcools.

Les dérivés des composés selon la présente invention peuvent également être des composés marqués par toute méthode convenable, par exemple à l'aide d'éléments radioactifs tels que $I^{125}$ ou $Tc^{99}$, ou bien en utilisant des composants fluorescents ou magnétiques. Grâce à ce type de marquage, les produits en cause peuvent être mis en évidence in vivo ou ex vivo.

Les dérivés des composés selon l'invention peuvent également être des conjugués avec des composés chimiques susceptibles d'améliorer leur activité ou qu'ils pourront amener au voisinage de sites particuliers, notamment du système immunitaire afin de leur permettre d'améliorer l'activité du produit chimique conjugué.

Un procédé d'obtention des composés de la présente invention est caractérisé en ce que l'on soumet le D.25 à une oxydation periodique puis à une réduction.

L'oxydation est préférentiellement mise en oeuvre à l'aide de métaperiodate de sodium. La réduction peut être mise en oeuvre à l'aide de $NaBH_4$, éventuellement en excès.

Les dérivés des composés peuvent être obtenus par des méthodes connues, méthode de marquage ou de conjugaison.

Les composés selon l'invention et leurs dérivés présentent des propriétés immunostimulantes remarquables et une absence de cytotoxicité. C'est pourquoi l'invention concerne également l'utilisation des composés et des dérivés à titre d'agent immunostimulant ainsi que des compositions pharmaceutiques comportant au moins un composé ou un dérivé conforme à la présente invention.

Les composés et surtout les dérivés marqués selon l'invention peuvent être utilisés également pour le diagnostic, en particulier pour la mise en évidence de certains éléments du système immunitaire.

D'autres caractéristiques et avantages de la présente invention apparaîtront à la lecture de la description détaillée qui va suivre.

Exemple 1 : Isolement du protéoglycane membranaire brut.

La biomasse de la souche de Klebsiella pneumoniae 145.I.IP est dispersée dans du tampon Tris-HCl (10 mM-pH 7,0) glacé contenant NaCl (0,15 M), puis soumise à un broyage mécanique destiné à rompre les parois cellulaires. Le lysat bactérien est clarifié par centrifugation continue à 15.000 g et le surnageant est recueilli. Le surnageant est traité par addition d'acide acétique jusqu'à pH 4,2, à froid, pour éliminer par précipitation les impuretés (acides nucléiques, grosses protéines). Le précipité d'impuretés est éliminé par centrifugation continue à 15.000 g. Le surnageant limpide est recueilli puis neutralisé par NaOH.

La solution est alors dialysée puis concentrée par ultrafiltration sur membrane coupant à 10.000 daltons.

La solution concentrée, obtenue à ce stade correspond au protéoglycane membranaire brut.

Exemple 2 : Isolement de la fraction polysaccharidique brute.

Cela consiste en une hydrolyse alcaline ménagée destinée à dépolymériser le protéoglycane membranaire brut pour libérer la fraction polysaccharidique. La solution concentrée et dialysée de protéoglycane membranaire brut obtenue précédemment est additionnée de NaOH pour avoir une concentration finale de 0,5 M en NaOH. On hydrolyse ensuite pendant 1 heure à 56°C. Après refroidissement rapide, la solution est neutralisée par HCl.

La solution neutralisée est clarifiée par filtration sur filtre-presse puis concentrée par ultrafiltration sur membrane coupant à 10 000 daltons.

Exemple 3 : Purification de la fraction polysaccharidique

Le concentrat obtenu à l'exemple précédent est soumis à un premier traitement enzymatique par le lysozyme, destiné à détruire les résidus de muréine pouvant subsister dans la préparation. l'hydrolyse est faite pendant 2 heures à température ambiante, en tampon Tris-HCL (10mM-pH 8,0) contenant : EDTA, $Na_2$ (4 mM) et 0,1 mg/ml de lysozyme.

Après action du lysozyme, les protéines contaminantes sont éliminées par protéolyse dans les conditions suivantes : le pH de la solution est ajusté à pH 7,0 puis on ajoute 0,1 mg/ml de protéinase K. On poursuit l'incubation 2 heures à 37°C sous agitation.

Le polysaccharide est ensuite isolé par précipitation alcoolique. Trois volumes d'alcool éthylique sont ajoutés à température ambiante. Après 30 minutes d'agitation, le précipité est recueilli par filtration. Le précipité est repris en solution dans l'eau distillée et la solution filtrée sur membrane de porosité 0,45 μm.

Les contaminants résiduels du polysaccharide provenant des hydrolyses enzymatiques sont éliminés par chromatographie de tamisage moléculaire sur une colonne industrielle Pharmacia moulée avec du gel SEPHACRYL S200 HR. Le volume d'échantillon déposé représente 5% du volume de gel. L'élution est faite avec de l'eau distillée à un débit linéaire de 5 cm/heure.

Le pic de polysaccharide purifié, détecté par mesure en continu de l'indice de réfraction est recueilli et concentré par ultrafiltration sur membrane coupant à 10 000 daltons, au 1/5è du volume initial.

La solution concentrée obtenue à ce stade correspond au polysaccharide purifié D.25.

Exemple 4 : Obtention du nouveau polysaccharide

La solution précédente est diluée afin d'obtenir 20 g de polysaccharide par litre de solution. On ajoute alors de l'acétate de sodium 0,1 M et le pH est ajusté à 3,8.

On ajoute ensuite 15 g de métaperiodate de sodium par litre de solution puis on maintient sous agitation pendant 48 heures à l'obscurité et à température 15°C.

L'excès de métaperiodate est ensuite éliminé par précipitation avec de l'hydroxyde de Baryum. Sous forme d'une solution concentrée ajoutée progressivement sous agitation jusqu'à fin de précipitation. Le précipité ainsi formé est éliminé par simple filtration.

Au filtrat précédent, on ajoute alors 21,6 g de $NaBH_4$ puis on laisse réagir pendant 18 heures à température ambiante. L'excès de $NaBH_4$ est ensuite détruit par addition d'acide acétique jusqu'à neutralité.

La solution obtenue est dialysée, concentrée sur membrane coupant à 10.000 Daltons puis lyophilisée. Le lyophilisat ainsi obtenu constitue le nouveau dérivé de D.25 de la présente invention.

Exemple 5 : Contrôle de l'absence de cytotoxicité

La cytotoxicité est mesurée par incubation in vitro de différentes concentrations du dérivé de la présente

3

invention dans une culture de cellules YAC-1 marquées au $^{51}$Cr. Après 4 heures d'incubation, le taux de $^{51}$Cr libéré par la lyse des cellules est mesuré dans le surnageant. On exprime les résultats par le pourcentage de cellules lysées par rapport à une culture témoin.

La lyse spontanée dans la culture témoin est d'environ 5%.

Les résultats sont répertoriés dans le Tableau I ci-dessous.

### TABLEAU I

| Concentration en µg/ml | % de lyse cellulaire |
|---|---|
| 0,05 | - 0,2 |
| 0,1 | 1,0 |
| 0,5 | 0,3 |
| 1,0 | - 1,0 |
| 5,0 | 0,3 |
| 10,0 | - 0,4 |
| 50,0 | - 0,1 |
| 100,0 | - 1,3 |

Les résultats montrent clairement l'absence totale de cytotoxicité du produit.

Exemple 6 : Activation des cellules NK in vitro

Des cellules effectrices (cellules spléniques de souris) sont préincubées pendant 6 heures dans un milieu RPMI-1640 contenant 5% de sérum foetal de veau avec différentes concentrations de dérivé à tester.

La mesure de l'activité NK est faite sur 10 000 cellules cibles YAC-1 marquées au $^{51}$Cr dans chaque puits, avec un rapport cellules effectrices/cellules cibles égal à 100/l.

Après 4 heures d'incubation, la quantité de $^{51}$Cr libérée dans le surnageant par la lyse des cellules est mesurée avec un compteur gamma.

Les résultats sont donnés dans le Tableau II ci-dessous

### TABLEAU II

| Concentration en µg/ml | % de lyse des cellules cibles YAC-1 |
|---|---|
| 0-témoin | 18,4 |
| 0,1 | 21,6 |
| 1,0 | 31,6 |
| 10 | 47,4 |
| 100 | 40,8 |

Les résultats confirment le très grand pouvoir stimulant du dérivé sur l'activité des cellules NK "in vitro".

Exemple 7 : Activation des cellules NK "in vitro" chez la souris. Comparaison avec le polysaccharide D.25.

Des souris CBA/H âgées de 6 à 8 semaines reçoivent une injection intrapéritonéale de 100 µg du dérivé de la présente invention un jour ou trois jours avant la mesure de l'activité de leurs cellules NK spléniques.

La mesure de l'activité NK est ensuite faite comme précédemment pour l'essai "in vitro".

Les résultats sont présentés dans le Tableau III ci-dessous

4

EP 0 288 382 B1

## TABLEAU III

| Produit injecté | % de lyse des cellules cibles | |
|---|---|---|
| | à J + 1 | à J + 3 |
| PBS témoins | 25,2 | 18,2 |
| D.25 (100 µg) | 20,0 | 22,0 |
| PS dérivé (100 µg) | 32,6 | 26,8 |

Les résultats présentés dans le Tableau montrent que le nouveau polysaccharide possède une capacité de stimulation des cellules NK supérieure à celle du D.25.

Cette activité se manifeste dès le premier jour après l'injection et se prolonge à J + 3 tandis que, dans les mêmes conditions, l'activité du D.25 n'apparaît qu'à J + 3.

## Revendications

1. Composé polysaccharidique caractérisé en ce qu'il est constitué par la répétition du monomère de structure :

$$\xrightarrow[1,3]{} \beta \; Gal_p \xrightarrow[1,3]{} \beta \; Ara \xrightarrow[1,3]{} \alpha \; Gal_p \xrightarrow[1,3]{} \beta \; Gal_p$$

$$\xrightarrow[1,3]{} \alpha \; Ara \xrightarrow[1,3]{} \alpha \; Gal_p \xrightarrow[1,3]{} \alpha \; Ara \xrightarrow[1,3]{} \beta \; Gal_p \xrightarrow[1,3]{} \alpha \; Gal_p$$

$$\xrightarrow[1,3]{} \beta \; Ara \xrightarrow[1,3]{} \ldots$$

dans lequel :

. $Gal_p$ signifie galactopyranose (sous forme $\alpha$ et $\beta$),

. Ara signifie arabinose (sous forme $\alpha$ et $\beta$).

2. Procédé de préparation du composé polysaccharidique selon la revendication 1 à partir du composé polysaccharidique dénommé D.25 extrait des protéoglycanes membranaires bactériens d'une souche de Klebsiella pneumonia déposée à la CNCM sous le N° 145-I-IP de poids moléculaire 30 ± 10 kd, tous les résidus galacto-furannose (Galf) par transformation de en arabinose par les étapes suivantes :

a) on soumet une solution du D.25 à une oxydation par du métapériodate de sodium en excès ;

b) on soumet le composé issu de a) à une réduction par du $NaBH_4$ en excès.

3. Utilisation du composé de la revendication 1 à titre d'agent immunostimulant.

4. Composition pharmaceutique caractérisée en ce qu'elle comprend à titre de principe actif un composé selon la revendication 1.

## Patentansprüche

1. Polysaccharid-Verbindung, dadurch gekennzeichnet, daß sie aus wiederkehrenden Monomer-Einheiten der Struktur

$$\begin{array}{c} \longrightarrow[1,3]{} \text{ß Gal}_p \xrightarrow[1,3]{} \text{ß Ara} \xrightarrow[1,3]{} \alpha \text{ Gal}_p \xrightarrow[1,3]{} \text{ß Gal}_p \xrightarrow[1,3]{} \alpha \text{ Ara} \\[2ex] \xrightarrow[1,3]{} \alpha \text{ Gal}_p \xrightarrow[1,3]{} \alpha \text{ Ara} \xrightarrow[1,3]{} \text{ß Gal}_p \xrightarrow[1,3]{} \alpha \text{ Gal}_p \xrightarrow[1,3]{} \\[2ex] \text{ß Ara} \xrightarrow[1,3]{} \quad \cdots \cdots \end{array}$$

besteht, in der

. Gal$_p$ Galactopyranose (in $\alpha$ und $\beta$ Form) bezeichnet, und

. Ara Arabinose (in $\alpha$ und $\beta$ Form) bezeichnet.

2. Verfahren zur Herstellung einer Polysaccharid-Verbindung gemäß Anspruch 1, ausgehend von einer als D.25 bezeichneten Polysaccharid-Verbindung, die aus bakteriellen membranären Proteoglycanen eines Stammes von <u>Klebsiella pneumonia</u>, mit einem Molekulargewicht von 30 ± 10 kd, hinterlegt bei CNCM unter der No. 145-I-IP, extrahiert wurde, durch Umwandlung aller Galacto-Furanose-Reste (Galf) in Arabinose gemäß der folgenden Stufen:

a) man unterzieht eine Lösung von D.25 einer Oxidation durch Natriummetaperiodat im Überschuß;

b) man unterzieht die aus a) stammende Verbindung einer Reduktion durch NaBH$_4$ im Überschuß.

3. Verwendung der Verbindung von Anspruch 1 als immunostimulierendes Mittel.

4. Pharmazeutische Zusammensetzung, dadurch gekennzeichnet, daß sie als Wirkstoff eine Verbindung gemäß Anspruch 1 umfaßt.

**Claims**

1. A polysaccharide compound characterized in that it is constituted by the repetition of the following monomer :

$$\begin{array}{c} \longrightarrow[1,3]{} \text{ß Gal}_p \xrightarrow[1,3]{} \text{ß Ara} \xrightarrow[1,3]{} \alpha \text{ Gal}_p \xrightarrow[1,3]{} \text{ß Gal}_p \\[2ex] \xrightarrow[1,3]{} \alpha \text{ Ara} \xrightarrow[1,3]{} \alpha \text{ Gal}_p \xrightarrow[1,3]{} \alpha \text{ Ara} \xrightarrow[1,3]{} \text{ß Gal}_p \xrightarrow[1,3]{} \alpha \text{ Gal}_p \\[2ex] \xrightarrow[1,3]{} \text{ß Ara} \xrightarrow[1,3]{} \end{array}$$

in which

. Gal$_p$ is galactopyranose (in the $\alpha$ and $\beta$ forms) AND

. Ara is arabinose (in the $\alpha$ and $\beta$ forms).

2. A process for obtaining a compound as claimed in claim 1, from the polysaccharidic compound called D.25 extracted from bacteriol membrane proteoglycanes of molecular weight of 30 ± 10 Kd of a <u>Klebsiella pneumonia</u> strain deposited to CNCM with n°145-I-IP, by transformation of all of the galacto-furannose residus (Galf) to arabinose which comprises :

a) subjecting D.25 to a periodate oxidation with a sodium metaperiodate in excess

b) subjecting the compound resulting from a) to a reduction with NaBH$_4$ in excess.

3. Use of a compound as claimed in any of claim as an immunostimulant.

4. A pharmaceutical composition which comprises, by way of the active principle, at least one compound as claimed in claim 1.